# EUROPÄISCHE PATENTSCHRIFT

(11) **EP 1 144 676 B1**
(45) Veröffentlichungstag und Bekanntmachung des Hinweises auf die Patenterteilung: **07.05.2003**
(21) Anmeldenummer: 00905030.3
(22) Anmeldetag: 07.02.2000
(51) Int. Cl.: C12Q 1/68

(54) **TESTKIT UND VERFAHREN ZUM QUANTITATIVEN NACHWEIS VON GENTECHNISCH VERÄNDERTER DNS IN LEBENSMITTELN MITTELS FLUORESZENZGEKOPPELTER PCR**
TEST KIT AND METHOD FOR QUANTITATIVELY DETECTING GENETICALLY MODIFIED DNA IN FOODSTUFF BY MEANS OF FLUORESCENCE-COUPLED PCR
KIT D'ESSAI ET PROCEDE DE DETECTION QUANTITATIVE DE DNS GENETIQUEMENT MODIFIE DANS DES PRODUITS ALIMENTAIRES PAR PCR COUPLE A LA FLUORESCENCE

(30) Priorität: 08.02.1999 DE 19906169
(43) Veröffentlichungstag der Anmeldung: 17.10.2001
(73) Patentinhaber: GeneScan Analytics GmbH, 79108 Freiburg (DE)
(72) Erfinder: LAUTER, Frank-Roman, D-14513 Teltow (DE); GROHMANN, Lutz, D-12161 Berlin (DE); STAESCHE, Roger, D-12159 Berlin (DE)
(74) Vertreter: Ziebig, Marlene, Dr. Dipl.-Chem.
(86) Internationale Anmeldenummer: EP0000935
(87) Internationale Veröffentlichungsnummer: WO00047764

(56) Entgegenhaltungen:
- WO-A-98/58083
- GIBSON U E M ET AL: "A NOVEL METHOD FOR REAL TIME QUANTITATIVE RT-PCR" GENOME RESEARCH,US,COLD SPRING HARBOR LABORATORY PRESS, Bd. 6, Nr. 10, 1. Oktober 1996 (1996-10-01), Seiten 995-1001, XP000642796 ISSN: 1088-9051
- DATABASE BIOSIS [Online] BIOSCIENCES INFORMATION SERVICE, PHILADELPHIA, PA, US; 1998 STUDER EDGAR ET AL: "Quantitative competitive PCR for the detection of genetically modified soybean and maize." Database accession no. PREV199800509818 XP002143861 in der Anmeldung erwähnt & ZEITSCHRIFT FUER LEBENSMITTEL-UNTERSUCHUNG UND -FORSCHUNG A, Bd. 207, Nr. 3, 1998, Seiten 207-213, ISSN: 1431-4630
- HEID C A ET AL: "REAL TIME QUANTITATIVE PCR" GENOME RESEARCH,US,COLD SPRING HARBOR LABORATORY PRESS, Bd. 6, Nr. 10, 1. Oktober 1996 (1996-10-01), Seiten 986-994, XP000642795 ISSN: 1088-9051 in der Anmeldung erwähnt
- SECCHIERO P ET AL: "QUANTITATIVE PCR FOR HUMAN HERPESVIRUSES 6 AND 7" JOURNAL OF CLINICAL MICROBIOLOGY,US,WASHINGTON, DC, Bd. 33, Nr. 8, 1. August 1995 (1995-08-01), Seiten 2124-2130, XP000564243 ISSN: 0095-1137
- WOUDENBERG T M ET AL: "QUANTITATIVE PCR BY REAL TIME DETECTION" PROCEEDINGS OF THE SPIE, 1996, XP000197422
- FREEMAN WILLARD M ET AL: "Quantitative RT-PCR: Pitfalls and potential." BIOTECHNIQUES, Bd. 26, Nr. 1, Januar 1999 (1999-01), Seiten 112-118, 120-122, 124-125, XP002143860 ISSN: 0736-6205

## Beschreibung

Die Erfindung betrifft einen Testkit und ein Verfahren zum qualitativen und quantitativen Nachweis von DNS in Lebensmitteln und Lebensmittelprodukten, die aus gentechnisch veränderten Organismen (GVO) stammt. Weiterhin betrifft die Erfindung die Verwendung spezieller Primer- und Sonden-DNS-Sequenzen für diesen Nachweis.

Die Herstellung von Lebensmitteln beinhaltet die Sicherstellung deren Qualität und die Information der Verbraucher über die Bestandteile und die Zusammensetzung von Lebensmittelprodukten.

Seit September 1998 gilt in Europa die Verordung Nr. 1139/98 des Rates der EG, nach der Lebensmittel gekennzeichet werden müssen, in denen DNS aus gentechnisch veränderten Organismen (GVO-DNS) enthalten ist. In derselben Verordung wird für die Zukunft die Festlegung eines unteren Schwellenwertes für die GVO-DNS-Menge empfohlen, ab welchem Lebensmittel gekennzeichnet werden müssen. Die Überprüfung der Verordnung in Bezug auf Nachweisbarkeit von GVO-DNS und Einhaltung des Schwellenwertes erfordert ein quantitatives GVO-DNS-Nachweissystem, das mit hoher Sensitivität und Reproduzierbarkeit arbeitet.

Der Nachweis von GVO-DNS wird zur Zeit mit Hilfe der Polymerase-Kettenreaktion (PCR, polymerase chain reaction) durchgeführt (Hupfer et al. 1997, Z Lebensm Unters Forsch 205: 442-445). Für den quantitativen Nachweis von GVO-DNS wird die Methode der quantitativen kompetitiven PCR angewendet (Studer et al. 1998, Z Lebensm Unters Forsch 207: 207-213).

Bei der quantitativen kompetitiven PCR werden die Target-Gene in Gegenwart von definierten Mengen an Standard-DNS amplifiziert. Die Standard-DNS wird von denselben Primern amplifiziert wie die Target-Gene. Die entstehenden PCR-Amplifikate für die Target-Gene und für die Standards unterscheiden sich in der Größe und lassen sich deshalb gel-elektrophoretisch auftrennen. Nach erfolgter PCR werden die PCR-Produkte angefärbt, gel-elektrophoretisch aufgetrennt, und die Mengen an Target-Gen-Amplifikaten und Standard-DNS-Amplifikaten werden densitrometrisch bestimmt. Da Standard-DNS-Kopien in definierten Mengen zugegeben werden, läßt sich die Menge an Target-Gen-Kopien durch den Vergleich von Standard-PCR-Produkten und Target-PCR-Produkten abschätzen.

So wird beispielsweise in WO 98/58083 eine RT-PCR mit Enzymrestriktion, Gelelektrophorese und anschließender Densitometriemessung beschrieben. In "Genome Research", Bd. 6, No. 10, 1996, S. 995-1001 wird eine "real time" quantitative RT-PCR offenbart. Eine interne Kontroll-Nukleinsäuresequenz wird im selben Gefäß und mit denselben Primern wie die Ziel-Nukleinsäuresequenz amplifiziert. Anschließend wird die amplifizierte Lösung auf 2 Gefäße aufgeteilt und ein. Gefäß mit einer fluoreszenzgekoppelten Sonde bebandelt, die mit der Ziel-Nukleinsäuresequenz hybridisiert. Die Lösung des anderen Gefäßes wird mit einer fluoreszenzgekoppelten Sonde behandelt, die mit der Kontroll-Nukleinsäuresequenz hybridisiert. Aus den gemessenen Werten kann die Menge der Ziel-Nukleinsäuresequenz bestimmt werden.

Obwohl sich mittels PCR gentechnisch veränderte DNS generell nachweisen läßt, und obwohl sich mittels quantitativer kompetitiver PCR deren Menge auch quantitativ bestimmen läßt, sind diese Methoden für die Routine-Analyse von Produkten, insbesondere von Lebensmitteln, schlecht geeignet. Die Methoden sind ungenügend automatisierbar, bergen die Gefahr von PCR-Produkt-Kontaminationen (falsch-positive Ergebnisse) und liefern Ergebnisse von ungenügender Genauigkeit.

Aufgabe der Erfindung war es, ein Vereinfachten Verfahren zum quantitativen Nachweis von gentechnisch veränderter DNS in Lebensmitteln bereitzustellen, mit dem eine kontaminationsfreie, hoch-automatisierbare und hoch reproduzierbare Analyse möglich ist.

Diese Aufgabe wird erfindungsgemäß gelöst durch die Anwendung der fluoreszenzgekoppelten PCR und den Einsatz spezieller Primer- und Sondenkombinationen in einer spezifischen Verfahrensvariante.

Bei der fluoreszenz-gekoppelten PCR handelt es sich um eine an sich bekannte Methode. So wird in US 5,210,015 und US 5,487,972 (TaqMan®) eine PCR-Reaktion beschrieben, an der drei Oligonukleotide beteiligt sind, zwei Primer und eine fluorogene Sonde. Die Sonde besteht aus einem Oligonukleotid, dessen 5'Ende mit einem fluoreszenten Reporter-Farbstoff (Fluoreszein-Derivat) markiert ist, während das 3' Ende einen Quencher-Farbstoff (Rhodamin-Derivat) trägt und außerdem mit einem Phosphatrest blockiert ist. Wird die intakte Sonde bei einer spezifischen Wellenlänge (488 nm) zur Fluoreszenz angeregt, so wird die Fluoreszenz des Reporter-Farbstoffes aufgrund der räumlichen Nähe zum Quencher durch einen Fluoreszenz-Energietransfer (FET) unterdrückt. Während der PCR hybridisiert die Sonde mit den Primern zunächst an den Matrizen-Strang der DNS. In der Extensionsphase trifft die Taq-Polymerase auf die Sonde, die zwischen den Primern positioniert ist und wird von der Exonuklease-Aktivität der Taq-Poymerase geschnitten. Durch die Sondenhydrolyse wird die räumliche Nähe - und damit auch der FET - zwischen Reporter und Quencher unterbrochen. Entsprechend der Akkumulation von PCR-Produkt steigt die Fluoreszenz des Reporters mit jedem PCR-Zyklus an. Das dabei gebildete Signal ist strikt sequenzspezifisch, da nicht 100% ig bindende Sondenmoleküle verdrängt werden, noch bevor die Exonukleaseaktivität der Taq Polymerase aktiviert ist. Die Veränderung der Fluoreszenzen der verschiedenen Farbstoffe kann schließlich mit Hilfe kommerzieller Geräte, z. B. ABIPRISM 7700 der Firma Perkin-Elmer Applied Biosystems Division (PEABD) im geschlossenen Reaktionsgefäß Zyklus für Zyklus erfaßt werden.

Der erfindungsgemäße Nachweis erfolgt nun, indem zunächst die Gesamt-DNS nach dem Fachmann an sich bekannten Methoden (vgl. Zimmermann et al, 1998, Z Lebensm Unters Forsch A 207 : 81-90) aus der Lebensmittelprobe extrahiert wird und
a) die Menge an Transgen in der Gesamt-DNS bestimmt wird, indem eine PCR-Reaktion mit einer Transgen-spezifischen fluoreszenzmarkierten Sonde S1 und zwei Transgen-spezifischen Primern P1 und P2 in einem ersten Reaktionsgefäß durchgeführt und die Änderung der Fluoreszenzstrahlung im Vergleich zur Kontrolle gemessen wird, wobei als interne Amplifikationskontrolle (IAK) für die Transgenbestimmung im ersten Reaktionsgefäß neben den Primern P1 und P2 und einer fluoreszenzmarkierten Sonde S2 ein synthetisches Genfragment eingesetzt wird (Target IAK DNS), das zwei Bindungsstellen für die Primer P1 und P2 und eine Bindungsstelle für die fluoreszenzmarkierte Sonde S2 besitzt, die sich sowohl in ihrer Sequenz von der Sonde S1 unterscheidet als auch mit einem anderen Fluoreszenzfarbstoff markiert ist als die Sonde S1,
b) ein Referenzgen ausgewählt und die Menge an Referenzgen in der Gesamt-DNS bestimmt wird, indem eine PCR-Reaktion mit einer Referenzgen-spezifischen fluoreszenzmarkierten Sonde S3 und zwei Referenzgen-spezifischen Primern P3 und P4 in einem zweiten Reaktionsgefäß durchgeführt und die Änderung der Fluoreszenzstrahlung im Vergleich zur Kontrolle gemessen wird, wobei als interne Amplifikationskontrolle (IAK) für die Referenzgen-Bestimmung im zweiten Reaktionsgefäß neben den Primern P3 und P4 und der fluoreszenzmarkierten Sonde S2 ein synthetisches Genfragment eingesetzt wird (Referenz IAK DNS), das zwei Bindungsstellen für die Primer P3 und P4 und eine Bindungsstelle für die fluoreszenzmarkierte Sonde S2 besitzt, die mit der fluoreszenzmarkierten Sonde S2 im Targetgen-System identisch ist, sich aber in der Sequenz und im Fluoreszenzfarbstoff von der Sonde S3 unterscheidet,
und letztlich aus dem Verhältnis der Menge an Transgen und der Menge an Referenzgen der Anteil an gentechnisch veränderter DNS berechnet wird.

Bei der fluoreszenz-gekoppelten PCR korreliert die Menge an untersuchten Genkopien mit einer gemessenen Fluoreszenzänderung im Vergleich zu einer Kontroll-PCR-Reaktion ohne DNS. Basierend auf der gemessenen Fluoreszenzänderung ist es damit möglich, auf die Menge an eingesetzter Proben-DNS zurückzurechnen.
Zur näheren Erläuterung der Erfindung ist nachfolgend definiert, was im Sinne der Erfindung unter einzelnen Begriffen zu verstehen ist:

### Primer

Primer sind DNS-Oligonukleotide. Unter den entsprechenden Bedingungen hybridisieren sie nur an die komplementäre DNS-Sequenz des zu detektierenden Genfragments. Sie dienen als Startpunkte für die Initiation der DNS-Synthese durch das Enzym DNS-Polymerase. Die Lage zweier Primer innerhalb eines Gens bestimmt, welches Genfragment mittels PCR vervielfältigt und nachfolgend detektiert werden kann.

### Sonden

Sonden (engl.: probe) sind DNS-Oligonucleotide, an die Reporter-Farbstoffe gekoppelt sind. Unter den entsprechenden Bedingungen hydrisieren sie nur an die komplementäre DNS-Sequenz des zu detektierenden Genfragments.

### Fluoreszenz-gekoppelte PCR

Bei der fluoreszenz-gekoppelten PCR sind die Sonden zwischen den Primern lokalisiert. In Gegenwart einer die Fluoreszenzstrahlung anregenden Lichtquelle führt eine erfolgreiche PCR-Reaktion zur Änderung der Sondenfluoreszenz. Die Änderung der Sondenfluoreszenzstrahlung korreliert mit der Menge an entstehenden PCR-Produkten und somit mit der Menge an ursprünglich untersuchten Genkopien. Anhand der Fluoreszenzstrahlung ist die Menge des zu detektierenden Gens kalkulierbar (Heid et al. 1996, Genome Methods 6: 986-994, Wittwer et al. 1997 BioTechniques 22: 130-138).

### Transgen

Als Transgen wird eine Nukleotidsequenz definiert, die vom Menschen manipuliert wurde, z. B. indem sie von einem Organismus in einen anderen Organismus, in dem sie natürlicherweise nicht vorhanden ist, transformiert wurde.

### Referenzgen

Als Referenzgen wird eine Nukleotidsequenz definiert, die sich in allen relevanten Varianten einer zu detektierenden Organismenart befindet. Das Referenzgen dient als Bezugsgröße bei der relativen Quantizierung von Transgenen. Die Menge an Referenzgen, somit deren Kopienzahl, wird bei der Quantifizierung als 100% definiert und die Kopienzahl des Transgens wird anschließend dazu in Beziehung gesetzt.

### Target-DNS

Target-DNS ist die Nukleotid-Sequenz, die von einem PCR-System erkannt und amplifiziert wird. Die Nukleotid-Sequenzen können verschiedenen Ursprungs sein. Beim Referenzgen handelt es um die genomische DNS eines bestimmten Genfragments, das in allen relevanten Varianten einer Art (z. B. Soja oder Mais) vorkommt. Beim Transgen handelt es sich um die gentechnisch veränderte DNS eines bestimmten DNS-Fragments, deren Existenz nachgewiesen werden soll. Bei den Target-IAK-DNS handelt es sich um erfindungsgemäße synthetische DNS-Fragmente, die einerseits die Nukleotidsequenzen der spezifischen Primer und andererseits die Nukleotidsequenz der erfindungsgemäßen universellen Sonde S2 beinhalten.

### Interne Amplifikationskontrolle (IAK)

Die interne Amplifikationskontrolle kontrolliert die Effizienz der PCR-Reaktion einer bestimmten Primerkombination. Sie besteht aus der erfindungsgemäßen Target-IAK-DNS bzw. der erfindungsgemäßen Referenz IAK DNS und der erfindungsgemäßen universellen Sonde S2.

Um die Menge an gentechnisch-veränderter DNS in Lebensmitteln bestimmen zu können, werden also erfindungsgemäß die folgenden Parameter gemessen:
(1) Es wird die Menge an Transgen in der Gesamt-DNS mit Hilfe der Primer P1 und P2 und der Sonde S1 gemessen. Diese Messung findet in Reaktionsgefäß (A) statt.
(2) Es wird die Menge an Referenzgen in der Gesamt-DNS mit Hilfe der Primer P3 und P4 und der Sonde S3 gemessen. Diese Messung findet in Reaktionsgefäß (B) statt.
   Das Verhältnis von Transgen zu Referenzgen ergibt den Anteil an gentechnisch veränderter DNS an der DNS einer bestimmten Organismenart; wobei die Menge an Referenzgen die Quantität der relevanten zu untersuchenden DNS reflektiert.
(3) In beiden Reaktionsgefäßen (A) und (B) wird mit Hilfe der Primer P1 bis P4, der Sonde S2 und den erfindungsgemäßen Target-IAK-DNS und Referenz-IAK-DNS die Effizienz der jeweiligen PCR-Reaktion gemessen.
   Die spezifischen internen Amplifikationskontrollen (IAK) bestehen aus den erfindungsgemäßen spezifischen Primern P1 und P2 für das Transgen und P3 und P4 für das Referenzgen, der erfindungsgemäßen universellen Sonde S2 und der erfindungsgemäßen Target-IAK-DNS für das Transgensystem und der erfindungsgemäßen Referenz-IAK-DNS für das Referenzgenszystem. Die IAKs ermöglichen die Kontrolle der DNS-Qualität. Dabei wird die PCR-Effizienz sowohl des Transgen- als auch des Referenzgensystems gemessen.
   Die Bereitstellung der vier erfindungsgemäßen Primer / Sondensysteme erlaubt die Kontrolle sowohl der DNS Quantität als auch deren Qualität und schafft damit die notwendige und hinreichende Voraussetzung für eine DNS-Quantifizierung.
   Erfindungsgemäß besteht die Möglichkeit, zwei PCR-Syteme pro Reaktionsgefäß parallel zu messen, da die Sonden für beide Systeme mit unterschiedlichen Fluoreszenzfarben markiert sind. Beispielsweise können die Sonden S1 und S3 mit dem gleichen Reporter-Farbstoff und S2 mit einem anderen Reporter-Farbstoff markiert sein.

In bevorzugten Ausführungsformen der Erfindung können so z. B. das Roundup Ready Soja-Gen (RRS-Gen) und das Bt-176 Mais-Gen als in Lebensmitteln häufig vorkommende Transgene nachgewiesen werden. Aber auch für andere Mais-Transgene, wie z. B. das Transgen im Bt-11-Mais, oder für den Nachweis des 35 S CMV Promoters als Screening-Verfahren zum unspezifischen Nachweis von GVO-DNS oder für alle zukünftig in den Lebensmittel-Markt eingeführten Transgene ist das erfindungsgemäße Nachweisverfahren einsetzbar und sehr gut geeignet.

In einer besonders bevorzugten Ausführungsform der Erfindung werden zum erfindungsgemäßen Nachweis des RRS-Gens, das die NS-Sequenz SEQ ID NO. 1 aufweist und nach Expertenschätzungen in 20000 bis 30000 Lebensmitteln enthalten ist, als Primer P1 die neuen Sequenzen SEQ ID NO. 3 oder SEQ ID NO. 3a und als Primer P2 die neuen Sequenzen SEQ ID NO. 4 oder SEQ ID NO. 4a oder deren durch Deletion, Substitution oder Addition erhaltene Varianten eingesetzt, die chemisch-synthetisch herstellbar sind. Als Sonde S1 hat sich die neue NS-Sequenz SEQ ID NO. 2 oder SEQ ID NO. 2a oder deren durch Deletion, Substitution oder Addition erhaltene Varianten als besonders geeignet erwiesen.

Die Herstellung kann ebenfalls auf chemisch-synthetischem Wege erfolgen.

Als Referenzgen für den erfindungsgemäßen Nachweis des RRS-Gens hat sich das Lektin-Gen als besonders geeignet erwiesen. Wird Lektin (vgl. SEQ ID NO. 11) als Referenzgen verwendet, so finden in einer bevorzugten Ausführungsform der Erfindung als Primer P3 und P4 die Sequenzen SEQ ID NO. 6 oder SEQ ID NO. 6a und SEQ ID NO. 7 oder deren durch Deletion, Substitution oder Addition erhaltene Varianten Anwendung. Als Sonde S3 hat sich die neue NS-Sequenz SEQ ID NO. 5 oder deren durch Deletion, Substitution oder Addition erhaltene Varianten als besonders geeignet erwiesen. Die Sonde S3 ist wie die Sonde S1 am 5'-Ende oder am 3'-Ende mit einem fluoreszenten Reporter-Farbstoff und am anderen Sequenzende mit einem Quencher markiert.

Erfindungsgemäß werden zur Transgen-Bestimmung und zur Referenzgenbestimmung interne Amplifikationskontrollen verwendet. Dabei haben sich für den Nachweis des RRS-Gens die NS-Sequenzen SEQ ID NO. 8 oder deren durch Deletion, Substitution oder Addition erhaltene Varianten als besonders geeignete interne Amplifikationskontrollen für die Transgenbestimmung (Target IAK DNS) und die NS-Sequenz SEQ ID NO. 10 oder SEQ ID NO. 10a oder deren durch Deletion, Substitution oder Addition erhaltene Varianten als besonders geeignete interne Amplifikationskontrollen für die Referenzgenbestimmung erwiesen.

In einer weiteren besonders bevorzugten Ausführungsform der Erfindung werden zum Nachweis des Bt-176 Mais-Gens, dessen Sequenz bekannt und in WO 93/07278 veröffentlicht ist, als Primer P1 die Sequenz SEQ ID NO. 13 und als Primer P2 die Sequenz SEQ No. 14 oder deren zu mindestens zu 80 % homologe Varianten eingesetzt. Als Sonde S1 hat sich die Sequenz SEQ ID NO. 12 oder deren zu mindestens zu 80 % homologe Varianten als besonders vorteilhaft erwiesen. Die Sonde S1 ist wie unten beschrieben mit einem Reporter-Farbstoff und mit einem Quencher markiert.

Als Referenzgen für den Nachweis des Bt-176 Mais-Gens dient erfindungsgemäß das Invertase-Gen des Mais, dessen Sequenz von Xu J., et al. in "Plant Physiol. 1995 Jul; 108(3):1293-4" veröffentlicht ist. Wird dieses Referenzgen verwendet, so finden in einer bevorzugten Ausführungsform der Erfindung als Primer P3 die Sequenz SEQ ID NO. 16 und als Primer P4 die Sequenz SEQ ID NO. 17 oder deren zu mindestens zu 80 % homologe Varianten Anwendung. Als referenzspezifische Sonde S3 dient hier vorzugsweise die Sequenz SEQ ID NO. 15 oder deren Varianten. Die Sonde S3 ist ebenfalls wie unten beschrieben markiert.

Als interne Amplifikationskontrollen dienen im erfindungsgemäßen Nachweisverfahren des Bt-176 Mais-Gens die Sequenz SEQ ID NO. 18 als Target IAK DNS und die Sequenz SEQ ID NO. 19 als Referenz IAK DNS bzw. deren zu mindestens zu 80 % homologe Sequenzen.

Als universelle Sonde S2 werden für alle Nachweise in einer bevorzugten Ausführungsform die NS-Sequenz SEQ ID NO. 9 oder deren durch Deletion, Substitution oder Addition erhaltene, zu mindestens zu 80 % homologe Varianten eingesetzt. Die Sonde S2 ist am 5'-Ende oder am 3'-Ende mit einem fluoreszenten Reporter-Farbstoff, vorzugsweise einem Fluoreszeinderivat ausgewählt aus 6-Carboxy-Fluoreszein, Tetrachloro-6-carboxy-Fluoreszein, 2,7-Dimethoxy-4,5-dichloro-6-carboxy-Fluoreszein, Hexachloro-6-carboxy-Fluoreszein markiert, der zu den Sonden S1 und S3 verschieden ist, beispielsweise mit Tetrachloro-6-carboxy-Fluoreszein. Am anderen Sequenzende ist die Sonde mit einem Quencher, vorzugsweise einem Rhodaminderivat, markiert, das mit dem der Sonden S1 und S3 identisch ist. Als besonders geeignet hat sich hierbei 6-Carboxytetramethylrhodamin erwiesen.

Die Sonden S1 und S3 sind am 5'-Ende oder am 3'-Ende ebenfalls mit einem fluoreszenten Reporter-Farbstoff, vorzugsweise einem Fluoreszeinderivat ausgewählt aus 6-Carboxy-Fluoreszein, Tetrachloro-6-carboxy-Fluoreszein, 2,7-Dimethoxy-4,5-dichloro-6-carboxy-Fluoreszein, Hexachloro-6-carboxy-Fluoreszein markiert. In einer ganz besonders bevorzugten Ausführungsform wird als Markierung 6-Carboxy-Fluoreszein (FAM) eingesetzt. Als Quencher am anderen Sequenzende findet vorzugsweise ein Rhodaminderivat, ganz besonders bevorzugt 6-Carboxytetramethylrhodamin, Anwendung.

Gegenstand der Erfindung sind nicht nur die in der Beschreibung und in den Ansprüchen konkret genannten Primer- und Sondensequenzen, sondern auch deren durch Deletion, Substitution oder Addition erhaltenen Varianten, sofern sie zu mindestens 80 %, vorzugsweise zu mindestens 90 %, zu der konkret genannten Sequenz homolog sind und eine ähnlich gute Testspezifität und Testsensitivität gewährleisten wie die konkret genannten Sequenzen.

Alle erfindungsgemäßen Primer- und Sondensequenzen können chemisch-synthetisch nach dem Fachmann gut bekannten Methoden hergestellt werden. Bis auf die RRS-Gen-Sequenz, die Lektin-Gen-Sequenz, die Bt-176-Gen-Sequenz, die Invertase-Gen-Sequenz und die Primer-Sequenz NO. 6 handelt es sich um neue Sequenzen. Die Sequenzen sind im Sequenzprotokoll, das Bestandteil der Beschreibung ist, dargestellt.

Mit dem erfindungsgemäßen hochspezifischen und hochsensitiven Verfahren ist es durch den Verfahrensaufbau und durch die speziellen Primer- und Sondenkombinationen möglich, erstmals für jede Produktuntersuchung die Genauigkeit der Untersuchung und die GVO-DNS-Nachweisgrenze anzugeben.

Gegenstand der Erfindung ist neben dem Nachweisverfahren auch der zugehörige Testkit zum Nachweis von gentechnisch veränderter DNS in Lebensmitteln, insbesondere des RRS-Gens und des Bt-176 Mais-Gens. Es finden vorzugsweise die beschriebenen Sequenzen der Sonden, Primer und internen Amplifikationskontrollen Anwendung. In einer besonders bevorzugten Ausführungsform wird als universelle Sonde S2 die Sequenz SEQ ID NO. 9 eingesetzt.

Der Testkit umfaßt erfindungsgemäß eine Transgen-spezifische fluoreszenmarkierte Sonde S1 und zwei Transgen-spezifische Primer P1 und P2, eine Referenzgen-spezifische fluoreszenzmarkierte Sonde S3 und zwei Referenzgen-spezifische Primer P3 und P4 sowie als interne Amplifikationskontrollen eine fluoreszenzmarkierte Sonde S2, die sich sowohl in ihrer Sequenz als auch in ihrer Fluoreszenzmarkierung von den Sonden S1 und S3 unterscheidet, ein synthetisches Genfragment (Target IAK DNS) mit zwei Bindungsstellen für die Primer P1 und P2 und einer Bindungsstelle für die Sonde S2 und ein synthetisches Genfragment (Referenz IAK DNS) mit zwei Bindungsstellen für die Primer P3 und P4 und einer Bindungsstelle für die Sonde S2.

Es hat sich gezeigt, daß sich der Nachweis von GVO-DNS auch durch Fluoreszenz-gekoppelte PCR mittels Hybridisierungssonden und dem erfindungsgemäßen Verfahrens aufbau und mit hoher Genauigkeit durchführen läßt. Dazu werden anstelle der einzelnen Sonde S1 und der einzelnen Sonde S3 jeweils zwei fluorogene Sonden eingesetzt. Das 3' Ende einer Sonde ist mit einem Donor-Farbstoff (Fluorescein-Derivat) markiert, während das 5' Ende der benachbarten Sonde mit einem Akzeptor-Farbstoff (z.B. Cy5, Light Cycler Red 640 oder Light Cycler Red 710) markiert ist. Die Nukleotidsequenzen der Sonden werden so gewählt, daß die Sonden in direkter Nachbarschaft mit der Target-DNS hybridisieren.

Dadurch entsteht eine direkte räumliche Nähe vom Donor-Farbstoff zum Akzeptorfarbstoff. Wird das Sondenpaar bei einer spezifischen Wellenlänge zur Fluoreszenz angeregt, so wird die Fluoreszenz des Donor-Farbstoffes auf den Akzeptorfarbstoff übertragen. Donor- und Akzeptorfarbstoffe werden so gewählt, daß sie bei verschiedenen Wellenlängen emitieren. Ein Anstieg der Emission des Akzeptorfarbstoffes kann nur gemessen werden, wenn nach Hydridisierung beider Sonden an die Target-DNS, die Anregungsenergie des Donorfarbstoffes auf den Akzeptorfarbstoff übertragen wurde. Je mehr PCR-Produkte entstehen, desto mehr Sondenpaare können hydridisieren, desto größer der Anstieg der Emissionstrahlung des Akzeptorfarbstoffes. Wie bei der TaqMan Technik ist die Menge an entstehender Fluoreszenzstrahlung proportional zur Menge an entstehenden PCR-Produkten und damit zur Menge an untersuchter Ausgangs-DNS.

Nachfolgend soll die Erfindung an Ausführungsbeispielen näher erläutert werden:

### Ausführungsbeispiel 1:

### Nachweis des RRS-Gens in Sojamehl SB2 der Fa. FLUKA (Deisenhofen)

Aus je 200 mg Sojamehl wurde Gesamt-DNS mittels CTAB-Methode isoliert (Zimmermann et al. 1998, Z Lebensm Unters Forsch A 207: 81-90). Die Ausbeute betrug etwa 500 µg DNS je 200 mg Sojamehl. Die isolierte DNS wurde in PCR-Reaktionen in Gegenwart des Trans-Gen-Systems oder des Referenz-Gen-Systems analysiert. Dazu wurden die im Folgenden beschriebenen Komponenten und Verfahren benutzt. Alle Komponenten, inklusive Primer und Sonden, wurden von der Firma PE Applied Biosystems Division, Weiterstadt, Deutschland, bezogen. Herstellung der TaqMan-PCR-Reaktionsgemische, Durchführung der PCR-Reaktion und Bedienung des PCR-Heizblocks bzw des Fluoreszenzdetektors (PEABD Model 7700 oder Model LS50B) erfolgte nach Anweisungen des Geräteherstellers (User's Manual, ABI Prism 7700 Sequence Detection System, PE Applied Biosystems Division, Foster City, USA 1997, bzw Users Manual, PE ABD LS50B).

Für die PCR-Amplifikation der isolierten DNS wurden folgende Komponenten in einem PCR Reaktionsgefäß für das Transgen-System bzw für das Referenz-Gen-System (PEABD Best.No. N80105080) gemischt. Es wurden PCR-Reaktionen von 50 µl Volumen pro Reaktion durchgeführt.

| Transgen-System (Roundup Ready System / RRS) | | |
|---|---|---|
| Reaktionskomponte | Volumen (µl) | Endkonzentration |
| DNS | 5, 000 | |
| H₂0 | 16,750 | |
| 10 x Puffer A | 5, 000 | 1 x |
| 25 mM MgCl₂ | 7, 000 | 3, 5 mM |
| 15 µM RRS Primer P1 | 2, 000 | 600 nM |
| 15 µM RRS Primer P2 | 2, 000 | 600 nM |
| 4 µM RRS Sonde S1 | 2, 000 | 160 nM |
| 2 µM IAK Sonde S2 | 3, 000 | 120 nM |
| dATP | 0, 400 | 200 µM |
| dCTP | 0, 400 | 200 µM |
| dGTP | 0, 400 | 200 µM |
| dUTP | 0, 800 | 400 µM |
| 5U/µl AmpliTaq Gold | 0, 250 | 0,025 U/µl |
| Polymerase | | |
| RRS IAK Target DNS (109 dil Plasmid) | 5, 000 | 100 Genkopien |
| Gesamtvolumen | 50,000 | |

| Referenz-Gen-System (Lektin System) | | |
|---|---|---|
| Reaktionskomponte | Volumen (µl) | Endkonzentration |
| DNS | 5, 000 | |
| H₂O | 14,750 | |
| 10 x Puffer A | 5, 000 | 1 x |
| 25 mM MgCl₂ | 11,000 | 5, 5 mM |
| 15 µM Lektin Primer P3 | 2, 000 | 600 nM |
| 15 µM Lektin Primer P4 | 2, 000 | 600 nM |
| 3 µM Lektin Sonde S3 | 2, 000 | 120 nM |
| 2 µM IAK Sonde S2 | 3, 000 | 120 nM |
| dATP | 0, 400 | 200 µM |
| dCTP | 0, 400 | 200 µM |
| dGTP | 0, 400 | 200 µM |
| dUTP | 0, 800 | 400 µM |
| 5U/µl AmpliTaq Gold Polymerase | 0, 250 | 0,025 U / µl |
| Lektin IAK Target DNS (109 dil Plasmid) | 3, 000 | 100 Genkopien |
| Gesamtvolumen | 50,000 | |

Folgendes Pipettierschema wurde zur Quantifizierung von Sojamehlproben angewendet (Reaktionsvolumen pro Reaktion: 50 µl):

### (i) NTC

Es wurden je 2 no template control (NTC) Reaktionen pro System (Transgen und Referenzgen) angestetzt. Diese Reaktionen enthalten ausser den IAKs keine DNS und ergeben in einem kontaminationsfreien Milieu keine Änderung der Reporter-fluoreszenzstrahlung der Sonden S1 und S3. Diese Negativkontrollen stellen sicher, daß keine falsch-positiven Ergebnisse erhalten werden.

### (ii) Standardgeraden

Es wurden je 3 Reaktionen von 4 verschiedenen Verdünnungen von 100% trangener Roundup Ready DNS hergestellt. Bei den Verdünnungen wurde 10 ng, 5 ng, 0.5 ng und 0. 25 ng DNS eingesetzt. Diese 12 Reaktionen wurden sowohl für das Transgen- als auch das Referenzgen-System nach o.g. Schema angesetzt. Zur Erstellung von 2 Standardgeraden, eine für das Transgen- und eine für das Referenzgen-System wurden also 24 PCR-Reaktionen angesetzt.

### (iii) Probenuntersuchung

Es wurden je 2 Reaktionen von 2 verschiedenen Verdünnungen der DNS hergestellt, die aus Sojamehl mit einem 2% Gehalt an Roundup Ready Soja extrahiert worden war. Bei den Verdünnungen wurde 10 ng und 5 ng DNS eingesetzt. Diese 4 Reaktionen wurden sowohl für das Transgen- als auch das Referenzgen-System nach o.g. Schema angesetzt. Zur Untersuchung der Proben-DNS wurden also 8 PCR-Reaktionen angesetzt.

Die insgesamt 36 Reaktionsgefäße wurden nach Abschluß des Pipettierarbeiten zur Durchführung der PCR-Reaktion in den Heizblock des Sequenz-Detektor PEABD Model 7700 überführt. Folgendes Temperatur-Schema wurde zur PCR-Amplifikation eingestellt:

| Temperatur-Bedingungen | | |
|---|---|---|
| 10 min | 95°C | Halten |
| 15 sec | 95°C | 45 |
| 60 sec | 60°C | Zyklen |
| 2 min | 25°C | Halten |

Der Sequenzdetektor PEABD Model 7700 wurde nach Anweisungen des Herstellers gestartet.
PCR-Bedingungen waren durch Variation des Primer- und Sondendesigns, der Primer-und Sondenkonzentration, der MgCl₂ Konzentration und der Oligonukleotid-Annealing Temperatur optimiert worden.
Während der PCR Reaktion wurde von dem Fluoreszenzdetektor des Sequenz-Detektor PEABD Model 7700 die Änderung der Fluoreszenzstrahlung gemessen.

Als Maß für die Menge an entstehenden PCR-Produkten und somit an eingesetzten Target-Genkopien wird bei der TaqMan Technologie der sogenannte 'Threshold cycle'werden. Die erhaltene Ct-Werte für IAK-positive Werte wurden mit Hilfe der Standardgeraden in relative Gen-Kopienzahlen umgerechnet. Das Verhältnis von relativer Transgen-Kopienzahl zu Referenz-Genkopienzahl ergibt den Anteil an GVO-DNS and der Gesamt-Soja-DNS der untersuchten Probe. Für die in diesem Beispiel untersuchte Sojamehl-Probe mit einem Anteil von 2% Roundup Ready Sojamehl FLUKA SB 2 Nr. 85478 ergab die Quantifizierung der Transgen-DNS versus Referenzgen-DNS tatsächlich 2, 00 % + / - 0, 30 %. Die Fehlerrechnung zur Bestimmung des Genaugigkeitsbereiches wurde gemäß User Bulletin #2, ABI Prism 7700 Sequence Detection System, 1997, S. 34 durchgeführt.
Das hier beschriebene System erfüllt somit voll die gestellte Aufgabe.

### Ausführungsbeispiel 2: Nachweis des Bt-176 Mais-Gens in Maismehl MZ 2 der Fa. FLUKA

In Analogie zum Ausführungsbeispiel 1 und unter Verwendung der Sequenzen ID NOs. 12-19 wie oben beschrieben wurde der Anteil an Bt-176 Mais-DNS in 200 mg Maismehl bestimmt. Für die in diesem Beispiel untersuchte Maismehl-Probe mit einem Anteil von 2% FLUKA Maismehl MZ 2 Nr. 63198 ergab die Quantifizierung der Transgen-DNS versus Referenzgen-DNS 1,91 ± 0,25%. Die Fehlerrechnung wurde wie in Beispiel 1 durchgeführt.

## Patentansprüche

1. Verfahren zum quantitativen Nachweis von gentechnisch veränderter DNS (Transgen) in Lebensmitteln,
**dadurch gekennzeichnet, daß**
der Nachweis mittels fluoreszenz-gekoppelter PCR erfolgt, dazu die Gesamt-DNS aus der Lebensmittelprobe extrahiert wird und
a) die Menge an Transgen in der Gesamt-DNS bestimmt wird, indem eine PCR-Reaktion mit einer Transgen-spezifischen fluoreszenzmarkierten Sonde S1 und zwei Transgen-spezifischen Primern P1 und P2 in einem ersten Reaktionsgefäß durchgeführt und die Änderung der Fluoreszenzstrahlung im Vergleich zur Kontrolle gemessen wird, wobei als interne Amplifikationskontrolle (IAK) für die Transgenbestimmung im ersten Reaktionsgefäß neben den Primern P1 und P2 und einer fluoreszenzmarkierten Sonde S2 ein synthetisches Genfragment eingesetzt wird (Target IAK DNS), das zwei Bindungsstellen für die Primer P1 und P2 und eine Bindungsstelle für die fluoreszenzmarkierte Sonde S2 besitzt, die sich sowohl in ihrer Sequenz von der Sonde S1 unterscheidet als auch mit einem anderen Fluoreszenzfarbstoff markiert ist als die Sonde S1,
b) ein Referenzgen ausgewählt und die Menge an Referenzgen in der Gesamt-DNS bestimmt wird, indem eine PCR-Reaktion mit einer Referenzgen-spezifischen fluoreszenzmarkierten Sonde S3 und zwei Referenzgen-spezifischen Primern P3 und P4 in einem zweiten Reaktionsgefäß durchgeführt und die Änderung der Fluoreszenzstrahlung im Vergleich zur Kontrolle gemessen wird, wobei als interne Amplifikationskontrolle (IAK) für die Referenzgen-Bestimmung im zweiten Reaktionsgefäß neben den Primern P3 und P4 und der fluoreszenzmarkierten Sonde S2 ein synthetisches Genfragment eingesetzt wird (Referenz IAK DNS), das zwei Bindungsstellen für die Primer P3 und P4 und eine Bindungsstelle für die fluoreszenzmarkierte Sonde S2 besitzt, die mit der fluoreszenzmarkierten Sonde S2 im Targetgen-System identisch ist, sich aber in der Sequenz und im Fluoreszenzfarbstoff von der Sonde S3 unterscheidet,
und letztlich aus dem Verhältnis der Menge an Transgen und der Menge an Referenzgen der Anteil an gentechnisch veränderter DNS berechnet wird.

2. Verfahren nach Anspruch 1,
**dadurch gekennzeichnet, daß**
das Roundup Ready Soja-Gen (RRS-Gen) als Transgen nachgewiesen wird.

3. Verfahren nach den Ansprüchen 1 und 2,
**dadurch gekennzeichnet, daß**
als Sonde S1 die Nukleinsäuresequenz SEQ ID NO. 2 oder SEQ ID NO. 2a oder deren durch Deletion, Substitution oder Addition erhaltene, zu mindestens 80 % homologe Varianten eingesetzt werden.

4. Verfahren nach den Ansprüchen 1 und 2,
**dadurch gekennzeichnet, daß**
als Primer P1 die Nukleinsäuresequenz SEQ ID NO. 3 oder SEQ ID NO. 3a und als Primer P2 die Nukleinsäuresequenz SEQ ID NO. 4 oder SEQ ID NO. 4a oder deren durch Deletion, Substitution oder Addition erhaltene, zu mindestens 80 % homologe Varianten eingesetzt werden.

5. Verfahren nach den Ansprüchen 1 und 2,
**dadurch gekennzeichnet, daß**
als Referenzgen das Lektin-Gen der SEQ ID No. 11 eingesetzt wird.

6. Verfahren nach den Ansprüchen 1, 2 und 5,
**dadurch gekennzeichnet, daß**
als Sonde S3 die Nukleinsäuresequenz SEQ ID NO. 5 oder deren durch Deletion, Substitution oder Addition erhaltene, zu mindestens 80 % homologe Varianten eingesetzt werden.

7. Verfahren nach den Ansprüchen 1, 2 und 5,
**dadurch gekennzeichnet, daß**
als Primer P3 die NS-Sequenz SEQ ID NO. 6 oder SEQ ID NO. 6a und als Primer P4 die NS-Sequenz SEQ ID NO. 7 oder deren durch Deletion, Substitution oder Addition erhaltene, zu mindestens 80 % homologe Varianten eingesetzt werden.

8. Verfahren nach den Ansprüchen 1 bis 4,
**dadurch gekennzeichnet, daß**
als synthetisches Genfragment der internen Amplifikationskontrolle für die Transgenbestimmung (Target IAK DNS) die NS-Sequenz SEQ ID NO. 8 oder SEQ ID NO. 8a oder deren durch Deletion, Substitution oder Addition erhaltene, zu mindestens 80 % homologe Varianten eingesetzt werden.

9. Verfahren nach den Ansprüchen 1 und 5 bis 7,
**dadurch gekennzeichnet, daß**
als synthetisches Genfragment der internen Amplifikationskontrolle für die Referenzgenbestimmung (Referenz IAK DNS) die NS-Sequenz SEQ ID NO. 10 oder SEQ ID NO. 10a oder deren durch Deletion, Substitution oder Addition erhaltene, zu mindestens 80 % homologe Varianten eingesetzt werden.

10. Verfahren nach Anspruch 1,
**dadurch gekennzeichnet, daß**
das Bt-176 Mais-Gen als Transgen nachgewiesen wird.

11. Verfahren nach den Ansprüchen 1 und 10,
**dadurch gekennzeichnet, daß**
als Sonde S1 die Sequenz SEQ ID No. 12 oder deren durch Deletion, Substitution oder Addition erhaltene, zu mindestens 80 % homologe Varianten eingesetzt werden.

12. Verfahren nach den Ansprüchen 1 und 10,
**dadurch gekennzeichnet, daß**
als Primer P1 die Sequenz SEQ ID No. 13 und als Primer P2 die Sequenz SEQ ID No. 14 oder deren durch Deletion, Substitution oder Addition erhaltene, zu mindestens 80 % homologe Varianten eingesetzt werden.

13. Verfahren nach den Ansprüchen 1 und 10,
**dadurch gekennzeichnet, daß**
als Referenzgen das Invertase-Gen des Maises eingesetzt wird.

14. Verfahren nach den Ansprüchen 1, 10 und 13,
**dadurch gekennzeichnet, daß**
als Sonde S3 die Sequenz SEQ ID No. 15 oder deren durch Deletion, Substitution oder Addition erhaltene, zu mindestens 80 % homologe Varianten eingesetzt werden.

15. Verfahren nach den Ansprüchen 1, 10 und 13,
**dadurch gekennzeichnet, daß**
als Primer P3 die Sequenz SEQ ID No. 16 und als Primer P4 die Sequenz SEQ ID No. 17 oder deren durch Deletion, Substitution oder Addition erhaltene, zu mindestens 80 % homologe Varianten eingesetzt werden.

16. Verfahren nach den Ansprüchen 1 und 10-12,
**dadurch gekennzeichnet, daß**
als Target IAK DNS die Sequenz SEQ ID No. 18 oder deren durch Deletion, Substitution oder Addition erhaltene, zu mindestens 80 % homologe Varianten eingesetzt werden.

17. Verfahren nach den Ansprüchen 1 und 13-15,
**dadurch gekennzeichnet, daß**
als Referenz IAK DNS die Sequenz SEQ ID No. 19 oder deren durch Deletion, Substitution oder Addition erhaltene, zu mindestens 80 % homologe Varianten eingesetzt werden.

18. Verfahren nach Anspruch 1,
**dadurch gekennzeichnet, daß**
als Sonde S2 die Nukleinsäure-Sequenz SEQ ID NO. 9 oder deren durch Deletion, Substitution oder Addition erhaltene, zu mindestens 80 % homologe Varianten eingesetzt werden.

19. Verfahren nach Anspruch 18,
**dadurch gekennzeichnet, daß**
die Sonde S2 am 5'-Ende oder am 3'-Ende mit einem fluoreszenten Reporter-Farbstoff, vorzugsweise einem Fluoreszeinderivat ausgewählt aus 6-Carboxy-Fluoreszein, Tetrachloro-6-carboxy-Fluoreszein, 2,7-Dimethoxy-4,5-dichloro-6-carboxy-Fluoreszein, Hexachloro-6-carboxy-Fluoreszein markiert ist, der verschieden zu dem der Sonden S1 und S3 ist, und die Sonde S2 am anderen Sequenzende mit einem Quencher, vorzugsweise einem Rhodaminderivat, markiert ist, das mit dem der Sonden S1 und S3 identisch ist, vorzugsweise mit TAMRA.

20. Verfahren nach Anspruch 1,
**dadurch gekennzeichnet, daß**
die Sonden S1 und S3 am 5'-Ende oder am 3'-Ende mit einem fluoreszenten Reporter-Farbstoff, vorzugsweise einem Fluoreszeinderivat ausgewählt aus 6-Carboxy-Fluoreszein, Tetrachloro-6-carboxy-Fluoreszein, 2,7-Dimethoxy-4,5-dichloro-6-carboxy-Fluoreszein, Hexachloro-6-carboxy-Fluoreszein markiert sind, vorzugsweise mit 6-Carboxy-Fluoreszein (FAM), und am jeweils anderen Ende mit einem Quencher, vorzugsweise einem Rhodaminderivat, vorzugsweise mit 6-Carboxytetramethylrhodamin (TAMRA), markiert sind.

21. Testkit zum quantitativen Nachweis von gentechnisch veränderter DNS (Transgen) in Lebensmitteln mittels fluoreszenz-gekoppelter PCR umfassend eine Transgen-spezifische fluoreszenmarkierte Sonde S1 und zwei Transgen-spezifische Primer P1 und P2, eine Referenzgen-spezifische fluoreszenzmarkierte Sonde S3 und zwei Referenzgen-spezifische Primer P3 und P4 sowie als interne Amplifikationskontrollen eine fluoreszenzmarkierte Sonde S2, die sich sowohl in ihrer Sequenz als auch in ihrer Fluoreszenzmarkierung von den Sonden S1 und S3 unterscheidet, ein synthetisches Genfragment (Target IAK DNS) mit zwei Bindungsstellen für die Primer P1 und P2 und einer Bindungsstelle für die Sonde 2 und ein synthetisches Genfragment (Referenz IAK DNS) mit zwei Bindungsstellen für die Primer P3 und P4 und einer Bindungsstelle für die Sonde S2.

22. Testkit nach Anspruch 21 zum Nachweis des RRS-Gens.

23. Testkit nach Anspruch 21 zum Nachweis des Bt-176 Mais Gens.

24. Testkit nach den Ansprüchen 21-23,
**dadurch gekennzeichnet, daß**
er als Sonde S2 die Sequenz SEQ ID NO. 9 oder deren zu mindestens 80 % homologe Varianten beinhaltet.

## Claims

1. A method of quantitatively detecting genetically modified DNA (transgene) in foodstuffs,
**characterized in that**
the detection is performed using fluorescence-coupled PCR, said method involving extracting the total DNA from the food sample, and
a) determining the amount of transgene in the total DNA by performing a PCR reaction with a transgene-specific fluorescence-labelled probe S1 and two transgene-specific primers P1 and P2 in a first reaction vessel and measuring the change in fluorescence radiation as compared to a control, a synthetic gene fragment (target IAC DNA) in addition to the primers P1 and P2 and a fluorescence-labelled probe S2 being used as internal amplification control (IAC) for transgene determination in the first reaction vessel, which fragment has two binding sites for the primers P1 and P2 and one binding site for the fluorescence-labelled probe S2 which not only differs from probe S1 in its sequence but also is labelled with a fluorescent dye other than that of probe S1;
b) selecting a reference gene and determining the amount of reference gene in the total DNA by performing a PCR reaction with a reference gene-specific fluorescence-labelled probe S3 and two reference gene-specific primers P3 and P4 in a second reaction vessel and measuring the change in fluorescence radiation as compared to a control, a synthetic gene fragment (reference IAC DNA) in addition to the primers P3 and P4 and the fluorescence-labelled probe S2 being used as internal amplification control (IAC) for reference gene determination in the second reaction vessel, which fragment has two binding sites for the primers P3 and P4 and one binding site for the fluorescence-labelled probe S2 which is identical to the fluorescence-labelled probe S2 in the target gene system, but differs from probe S3 with respect to sequence and fluorescent dye; and
ultimately, calculating the level of genetically modified DNA from the ratio of the amounts of transgene and reference gene.

2. The method according to claim 1, **characterized in that** the Roundup Ready soy gene (RRS gene) is detected as transgene.

3. The method according to claims 1 and 2, **characterized in that** the nucleic acid sequence SEQ ID NO. 2 or SEQ ID NO. 2a or variants thereof obtained by deletion, substitution or addition and having at least 80% homology are used as probe S1.

4. The method according to claims 1 and 2, **characterized in that** the nucleic acid sequence SEQ ID NO. 3 or SEQ ID NO. 3a and the nucleic acid sequence SEQ ID NO. 4 or SEQ ID NO. 4a or the variants thereof obtained by deletion, substitution or addition and having at least 80% homology are used as primer P1 and primer P2, respectively.

5. The method according to claims 1 and 2, **characterized in that** the lectin gene having the SEQ ID No. 11 is used as reference gene.

6. The method according to claims 1, 2 and 5, **characterized in that** the nucleic acid sequence SEQ ID NO. 5 or variants thereof obtained by deletion, substitution or addition and having at least 80% homology are used as probe S3.

7. The method according to claims 1, 2 and 5, **characterized in that** the NA sequence SEQ ID NO. 6 or SEQ ID NO. 6a and the NA sequence SEQ ID NO. 7 or the variants thereof obtained by deletion, substitution or addition and having at least 80% homology are used as primer P3 and primer P4, respectively.

8. The method according to claims 1 to 4, **characterized in that** the NA sequence SEQ ID NO. 8 or SEQ ID NO. 8a or variants thereof obtained by deletion, substitution or addition and having at least 80% homology are used as synthetic gene fragment of the internal amplification control for transgene determination (target IAC DNA).

9. The method according to claims 1 and 5 to 7, **characterized in that** the NA sequence SEQ ID NO. 10 or SEQ ID NO. 10a or variants thereof obtained by deletion, substitution or addition and having at least 80% homology are used as synthetic gene fragment of the internal amplification control for reference gene determination (reference IAC DNA).

10. The method according to claim 1, **characterized in that** the Bt-176 maize gene is detected as transgene.

11. The method according to claims 1 and 10, **characterized in that** the sequence SEQ ID NO. 12 or variants thereof obtained by deletion, substitution or addition and having at least 80% homology are used as probe S1.

12. The method according to claims 1 and 10, **characterized in that** the sequence SEQ ID NO. 13 and the sequence SEQ ID NO. 14 or variants thereof obtained by deletion, substitution or addition and having at least 80% homology are used as primer P1 and primer P2, respectively.

13. The method according to claims 1 and 10, **characterized in that** the invertase gene of maize is used as reference gene.

14. The method according to claims 1, 10 and 13, **characterized in that** the sequence SEQ ID NO. 15 or variants thereof obtained by deletion, substitution or addition and having at least 80% homology are used as probe S3.

15. The method according to claims 1, 10 and 13, **characterized in that** the sequence SEQ ID NO. 16 and the sequence SEQ ID NO. 17 or variants thereof obtained by deletion, substitution or addition and having at least 80% homology are used as primer P3 and primer P4, respectively.

16. The method according to claims 1 and 10 - 12, **characterized in that** the sequence SEQ ID NO. 18 or variants thereof obtained by deletion, substitution or addition and having at least 80% homology are used as target IAC DNA.

17. The method according to claims 1 and 13 - 15, **characterized in that** the sequence SEQ ID NO. 19 or variants thereof obtained by deletion, substitution or addition and having at least 80% homology are used as reference IAC DNA.

18. The method according to claim 1, **characterized in that** the nucleic acid sequence SEQ ID NO. 9 or variants thereof obtained by deletion, substitution or addition and having at least 80% homology are used as probe S2.

19. The method according to claim 18, **characterized in that** the probe S2 at its 5' end or its 3' end is labelled with a reporter fluorescent dye, preferably a fluorescein derivative selected from 6-carboxyfluorescein, tetrachloro-6-carboxyfluorescein, 2,7-dimethoxy-4,5-dichloro-6-carboxyfluorescein, hexachloro-6-carboxyfluorescein, which dye is different from that of probes S1 and S3, and at its other sequence end, the probe S2 is labelled with a quencher, preferably a rhodamine derivative identical to that of probes S1 and S3, preferably with TAMRA.

20. The method according to claim 1, **characterized in that** the probes S1 and S3 are labelled at their 5' end or at their 3' end with a reporter fluorescent dye, preferably a fluorescein derivative selected from 6-carboxyfluorescein, tetrachloro-6-carboxyfluorescein, 2,7-dimethoxy-4,5-dichloro-6-carboxyfluorescein, hexachloro-6-carboxyfluorescein, preferably with 6-carboxyfluorescein (FAM), and at each of their other ends with a quencher, preferably a rhodamine derivative, preferably with 6-carboxytetramethylrhodamine (TAMRA).

21. A test kit for the quantitative detection of genetically modified DNA (transgene) in foods using fluorescence-coupled PCR, comprising a transgene-specific, fluorescence-labelled probe S1 and two transgene-specific primers P1 and P2, a reference gene-specific, fluorescence-labelled probe S3, and two reference gene-specific primers P3 and P4, as well as a fluorescence-labelled probe S2 as internal amplification control, which probe differs from probes S1 and S3 both in its sequence and its fluorescence-labelling, a synthetic gene fragment (target IAC DNA) having two binding sites for the primers P1 and P2 and one binding site for the probe S2, and a synthetic gene fragment (reference IAC DNA) having two binding sites for the primers P3 and P4 and one binding site for the probe S2.

22. The test kit according to claim 21, used in the detection of the RRS gene.

23. The test kit according to claim 21, used in the detection of the Bt-176 maize gene.

24. The test kit according to claims 21-23, **characterized in that** the kit includes as probe S2 the sequence SEQ ID NO. 9 or variants thereof having at least 80% homology.

## Revendications

1. Procédé d'identification quantitative d'ADN génétiquement modifié (transgène) dans les aliments, **caractérisé en ce que**
l'identification s'effectue par PCR couplée à la fluorescence, qu'à cet effet on extrait l'ADN total de l'échantillon d'aliment et
a) que la quantité de transgène dans l'ADN total est déterminée en effectuant une réaction PCR avec une sonde S1 marquée en fluorescence spécifique au transgène et deux amorces P1 et P2 spécifiques au transgène dans un premier réacteur et que l'on mesure la modification du rayonnement fluorescent en comparaison d'un témoin, en utilisant, en tant que témoin interne d'amplification (IAK) pour identifier le transgène dans le premier réacteur, en plus des amorces P1 et P2 et d'une sonde S2 marquée en fluorescence, un fragment de gène synthétique (ADN témoin d'amplification interne cible), qui comporte deux sites de liaison pour les amorces P1 et P2 et un site de liaison pour la sonde S2 marquée en fluorescence, qui se différencie par sa séquence de la sonde S1 et qui est marquée avec un indicateur de fluorescence différent de celui de la sonde S1,
b) qu'on sélectionne un gène de référence et on détermine la quantité de gène de référence dans l'ADN total en effectuant une réaction PCR avec une sonde S3 marquée en fluorescence spécifique au gène de référence et deux amorces P3 et P4 spécifiques au gène de référence dans un second réacteur et que l'on mesure la modification du rayonnement fluorescent en comparaison d'un témoin, en utilisant, en tant que témoin interne d'amplification (IAK) pour identifier le gène de référence dans le second réacteur, en plus des amorces P3 et P4 et de la sonde S2 marquée en fluorescence, un fragment de gène synthétique (ADN témoin d'amplification interne de référence), qui comporte deux sites de liaison pour les amorces P3 et P4 et un site de liaison pour la sonde S2 marquée en fluorescence, qui est identique à la sonde S2 utilisée dans le système cible, mais qui se différencie par sa séquence et son indicateur de fluorescence de la sonde S3,
et qu'enfin on calcule la proportion d'ADN génétiquement modifié à partir du rapport de la quantité de transgène à la quantité de gène de référence.

2. Procédé selon la revendication 1,
**caractérisé en ce**
**qu'**en tant que transgène, on identifie le gène de soja roundup ready (gène RRS).

3. Procédé selon les revendications 1 et 2,
**caractérisé en ce**
**qu'**en tant que sonde S1, on utilise la séquence d'acides nucléiques SEQ ID N° 2 ou SEQ ID N° 2a ou leurs variantes homologues au moins à 80% obtenues par délétion, substitution ou addition.

4. Procédé selon les revendications 1 et 2,
**caractérisé en ce**
**qu'**en tant qu'amorce P1, on utilise la séquence d'acides nucléiques SEQ ID N° 3 ou SEQ ID N° 3a et qu'en tant qu'amorce P2, on utilise la séquence d'acides nucléiques SEQ ID N° 4 ou SEQ ID N° 4a ou leurs variantes homologues au moins à 80% obtenues par délétion, substitution ou addition.

5. Procédé selon les revendications 1 et 2,
**caractérisé en ce**
**qu'**en tant que gène de référence, on utilise le gène de lectine de SEQ ID N° 11.

6. Procédé selon les revendications 1, 2 et 5,
**caractérisé en ce**
**qu'**en tant que sonde S3, on utilise la séquence d'acides nucléiques SEQ ID N° 5 ou ses variantes homologues au moins à 80% obtenues par délétion, substitution ou addition.

7. Procédé selon les revendications 1, 2 et 5,
**caractérisé en ce**
**qu'**en tant qu'amorce P3, on utilise la séquence d'acides nucléiques SEQ ID N° 6 ou SEQ ID N° 6a et qu'en tant qu'amorce P4, on utilise la séquence d'acides nucléiques SEQ ID N° 7 ou leurs variantes homologues au moins à 80% obtenues par délétion, substitution ou addition.

8. Procédé selon les revendications 1 à 4,
**caractérisé en ce**
**qu'**en tant que fragment de gène synthétique témoin d'amplification interne pour la détermination du transgène (ADN témoin d'amplification interne cible), on utilise la séquence d'acides nucléiques SEQ ID N°8 ou SEQ ID N° 8a, ou leurs variantes homologues au moins à 80% obtenues par délétion, substitution ou addition.

9. Procédé selon les revendications 1 et de 5 à 7,
**caractérisé en ce**
**qu'**en tant que fragment de gène synthétique servant de témoin d'amplification interne pour la détermination du gène de référence (ADN témoin d'amplification interne de référence), on utilise la séquence d'acides nucléiques SEQ ID N°10 ou SEQ ID N° 10a, ou leurs variantes homologues au moins à 80% obtenues par délétion, substitution ou addition.

10. Procédé selon la revendication 1,
**caractérisé en ce**
**qu'**en tant que transgène, on identifie le gène de maïs Bt-176.

11. Procédé selon les revendications 1 et 10,
**caractérisé en ce**
**qu'**en tant que sonde S1, on utilise la séquence SEQ ID N° 12 ou ses variantes homologues à au moins 80% obtenues par délétion, substitution ou addition.

12. Procédé selon les revendications 1 et 10,
**caractérisé en ce**
**qu'**en tant qu'amorce P1, on utilise la séquence SEQ ID N° 13 et qu'en tant qu'amorce P2, on utilise la séquence SEQ ID N°14 ou leurs variantes homologues à au moins 80% obtenues par délétion, substitution ou addition.

13. Procédé selon les revendications 1 et 10,
**caractérisé en ce**
**qu'**en tant que gène de référence, on utilise le gène d'invertase du maïs.

14. Procédé selon les revendications 1, 10 et 13,
**caractérisé en ce**
**qu'**en tant que sonde S3, on utilise la séquence SEQ ID N° 15 ou ses variantes homologues au moins à 80% obtenues par délétion, substitution ou addition.

15. Procédé selon les revendications 1, 10 et 13,
**caractérisé en ce**
**qu'**en tant qu'amorce P3, on utilise la séquence SEQ ID N° 16 et qu'en tant qu'amorce P4, on utilise la séquence SEQ ID N° 17 ou leurs variantes homologues à au moins 80% obtenues par délétion, substitution ou addition.

16. Procédé selon les revendications 1 et de 10 à 12,
**caractérisé en ce**
**qu'**en tant qu'ADN témoin d'amplification interne cible, on utilise la séquence SEQ ID N°18, ou ses variantes homologues à au moins 80% obtenues par délétion, substitution ou addition.

17. Procédé selon les revendications 1 et de 13 à 15,
**caractérisé en ce**
**qu'**en tant qu'ADN témoin d'amplification interne de référence, on utilise la séquence SEQ ID N°19, ou ses variantes homologues à au moins 80% obtenues par délétion, substitution ou addition.

18. Procédé selon la revendication 1,
**caractérisé en ce**
**qu'**en tant que sonde S2, on utilise la séquence d'acides nucléiques SEQ ID N° 9 ou ses variantes homologues à au moins 80% obtenues par délétion, substitution ou addition.

19. Procédé selon la revendication 18,
**caractérisé en ce que**
la sonde S2 est marquée à l'extrémité 5' ou à l'extrémité 3' avec un colorant reporter fluorescent, de préférence un dérivé de la fluorescéine sélectionné entre la 6-carboxyfluorescéine, la tétrachloro-6-carboxyfluorescéine, la 2,7-diméthoxy-4,5-dichloro-6-carboxyfluorescéine, l'hexachloro-6-carboxyfluorescéine, qui est différent de celui utilisé pour les sondes S1 et S3, et **en ce que** la sonde S2 est marquée à l'autre extrémité de la séquence avec un agent d'extinction, de préférence un dérivé de la rhodamine, qui est identique à celui utilisé pour les sondes S1 et S3, de préférence du TAMRA.

20. Procédé selon la revendication 1,
**caractérisé en ce que**
les sondes S1 et S3 sont marquées à l'extrémité 5' ou à l'extrémité 3' avec un colorant reporter fluorescent, de préférence un dérivé de la fluorescéine sélectionné entre la 6-carboxyfluorescéine, la tétrachloro-6-carboxyfluorescéine, la 2,7-diméthoxy-4,5-dichloro-6-carboxyfluorescéine, l'hexachloro-6-carboxyfluorescéine, de préférence avec de la 6-carboxyfluorescéine (FAM), et **en ce qu'**elles sont marquées respectivement à l'autre extrémité de la séquence avec un agent d'extinction, de préférence un dérivé de la rhodamine, préférentiellement avec de la 6-carboxytétraméthylrhodamine (TAMRA).

21. Kit d'essai servant à l'identification quantitative de l'ADN génétiquement modifié (transgène) dans les aliments par PCR couplée à la fluorescence comportant une sonde S1 marquée en fluorescence spécifique au transgène et deux amorces P1 et P2 spécifiques au transgène, une sonde S3 marquée en fluorescence spécifique au gène de référence et deux amorces P3 et P4 spécifiques au gène de référence ainsi qu'une sonde S2 marquée en fluorescence servant de témoin d'amplification interne, qui se différencie des sondes S1 et S3 à la fois par sa séquence et son marquage fluorescent, un fragment de gène synthétique (ADN témoin d'amplification interne cible) possédant deux sites de liaison pour les amorces P1 et P2 et un site de liaison pour la sonde S2 et un fragment de gène synthétique (ADN témoin d'amplification interne de référence) possédant deux sites de liaison pour les amorces P3 et P4 et un site de liaison pour la sonde S2.

22. Kit d'essai selon la revendication 21 servant à l'identification du gène RRS.

23. Kit d'essai selon la revendication 21 servant à l'identification du gène de maïs Bt-176.

24. Kit d'essai selon les revendications de 21 à 23,
**caractérisé en ce**
**qu'**il contient, en tant que sonde S2, la séquence SEQ ID N° 9 ou ses variantes homologues à au moins 80%.


### SEQUENZPROTOKOLL

<110> BioInside Gesellschaft für Biodiagnostik, Auftragsforschung und Consulting mbH

<120> Testkit und Verfahren zum quantitativen Nachweis von gentechnisch veränderter DNS in Lebensmitteln mittels fluoreszenzgekoppelter PCR

<130> PCT/EP00/
<140> PCT/EP00/
<141> 2000-02-07

<150> DE 199 06 169.6
<151> 1999-02-08

<160> 25

<170> PatentIn Ver. 2.1

<210> 1
<211> 240
<212> DNA
<213> Künstliche Sequenz

<220>
<223> Beschreibung der künstlichen Sequenz: Roundup Soja-Gen (RRS-Gen)

<400> 1
   gtcttcaaag caagtggatt gatgtgatat ctccactgac gtaagggatg acgcacaatc 60
   ccactatcct tcgcaagacc cttcctctat ataaggaagt tcatttcatt tggagaggac 120
   acgctgacaa gctgactcta gcagatcttt caagaatggc acaaattaac aacatggctc 180
   aagggataca aacccttaat cccaattcca atttccataa accccaagtt cctaaatctt 240

<210> 2
<211> 24
<212> DNA
<213> Künstliche Sequenz

<220>
<223> Beschreibung der künstlichen Sequenz: Sonde

<400> 2
   caagctgact ctagcagatc tttc 24

<210> 2a
<211> 24
<212> DNA
<213> Künstliche Sequenz

<220>
<223> Beschreibung der künstlichen Sequenz: Sonde

<400> 2a

<210> 3
<211> 21
<212> DNA
<213> Künstliche Sequenz

<220>
<223> Beschreibung der künstlichen Sequenz: Primer

<400> 3
   catttggaga ggacacgctg a 21

<210> 3a
<211> 21
<212> DNA
<213> Künstliche Sequenz

<220>
<223> Beschreibung der künstlichen Sequenz: Primer

<400> 3a

<210> 4
<211> 23
<212> DNA
<213> Künstliche Sequenz

<220>
<223> Beschreibung der künstlichen Sequenz: Primer

<400> 4
   gggtttgtat cccttgagcc atg 23

<210> 4a
<211> 24
<212> DNA
<213> Künstliche Sequenz

<220>
<223> Beschreibung der künstlichen Sequenz: Primer

<400> 4a

<210> 5
<211> 24
<212> DNA
<213> Künstliche Sequenz

<220>
<223> Beschreibung der künstlichen Sequenz: Sonde

<400> 5
   cttcaccttc tatgcccctg acac 24

<210> 6
<211> 22
<212> DNA
<213> Künstliche Sequenz

<220>
<223> Beschreibung der künstlichen Sequenz: Primer

<400> 6
   gccctctact ccacccccat cc 22

<210> 6a
<211> 21
<212> DNA
<213> Künstliche Sequenz

<220>
<223> Beschreibung der künstlichen Sequenz: Primer

<400> 6a

<210> 7
<211> 23
<212> DNA
<213> Künstliche Sequenz

<220>
<223> Beschreibung der künstlichen Sequenz: Primer

<400> 7
   gaaggcaagc ccatctgcaa gcc 23

<210> 8
<211> 142
<212> DNA
<213> Künstliche Sequenz

<220>
<223> Beschreibung der künstlichen Sequenz: Target IAK DNS für das RRS-Gen

<400> 8
   catttggaga ggacacgctg aggacgttcg ccaattttcg cctcccacgt ctcaccgagc 60
   gtggtgttta cgaaggtttt acgttttccc gtatcccctt tcgttttcat ccagtctttc 120
   atggctcaag ggatacaaac cc 142

<210> 8a
<211> 143
<212> DNA
<213> Künstliche Sequenz

<220>
<223> Beschreibung der künstlichen Sequenz: Target IAK DNS für das RRS-Gen

<400> 8a

<210> 9
<211> 21
<212> DNA
<213> Künstliche Sequenz

<220>
<223> Beschreibung der künstlichen Sequenz: Sonde

<400> 9
   tcgcctccca cgtctcaccg a 21

<210> 10
<211> 150
<212> DNA
<213> Künstliche Sequenz

<220>
<223> Beschreibung der künstlichen Sequenz: Referenz IAK DNS für das RRS-Gen

<400> 10
   gccctctact ccacccccat ccggacgttc gccaattttc gcctcccacg tctcaccgag 60
   cgtggtgttt acgaaggttt tacgttttcc cgtatcccct ttcgttttca tccagtcttt 120
   gacaatcggc ttgcagatgg gcttgccttc 150

<210> 10a
<211> 149
<212> DNA
<213> Künstliche Sequenz

<220>
<223> Beschreibung der künstlichen Sequenz: Referenz IAK DNS für das RRS-Gen

<400> 10a

<210> 11
<211> 250
<212> DNA
<213> Künstliche Sequenz

<220>
<223> Beschreibung der künstlichen Sequenz: Lektin-Gen

<400> 11
   gggaaagtta caactcaata aggttgacga aaacggcacc ccaaaaccct cgtctcttgg 60
   tcgcgccctc tactccaccc ccatccacat ttgggacaaa gaaaccggta gcgttgccag 120
   cttcgccgct tccttcaact tcaccttcta tgcccctgac acaaaaaggc ttgcagatgg 180
   gcttgccttc tttctcgcac caattgacac taagccacaa acacatgcag gttatcttgg 240
   tcttttcaac                                                        250

<210> 12
<211> 21
<212> DNA
<213> Künstliche Sequenz

<220>
<223> Beschreibung der künstlichen Sequenz: Sonde

<400> 12
   atgtccacca ggcccagcac g                                           21

<210> 13
<211> 21
<212> DNA
<213> Künstliche Sequenz

<220>
<223> Beschreibung der künstlichen Sequenz: Primer

<400> 13
   cccatcgaca tcagcctgag c                                           21

<210> 14
<211> 20
<212> DNA
<213> Künstliche Sequenz

<220>
<223> Beschreibung der künstlichen Sequenz: Primer

<400> 14
   caggaaggcg tcccactggc                                             20

<210> 15
<211> 23
<212> DNA
<213> Künstliche Sequenz

<220>
<223> Beschreibung der künstlichen Sequenz: Sonde

<400> 15
   ccacctcttc taccagtgga acc                                         23

<210> 16
<211> 21
<212> DNA
<213> Künstliche Sequenz

<220>
<223> Beschreibung der künstlichen Sequenz: Primer

<400> 16
   cgctgtatca caagggctgg t 21

<210> 17
<211> 20
<212> DNA
<213> Künstliche Sequenz

<220>
<223> Beschreibung der künstlichen Sequenz: Primer

<400> 17 agcggtaggt gcagccagtg

<210> 18
<211> 136
<212> DNA
<213> Künstliche Sequenz

<220>
<223> Beschreibung der künstlichen Sequenz: Target IAK DNS für das Bt-176 Mais-Gen

<400> 18
   cccatcgaca tcagcctgag cgttcgccaa ttttcgcctc ccacgtctca ccgagcgtgg 60
   tgtttacgaa ggttttacgt tttcccgtat cccctttcgt tttcatccag tcttttgcca 120
   gtgggacgcc ttcctg 136

<210> 19
<211> 136
<212> DNA
<213> Künstliche Sequenz

<220>
<223> Beschreibung der künstlichen Sequenz: Referenz IAK DNS für das Bt-176 Mais-Gen

<400> 19
   cgctgtatca caagggctgg tgttcgccaa ttttcgcctc ccacgtctca ccgagcgtgg 60
   tgtttacgaa ggttttacgt tttcccgtat cccctttcgt tttcatccag tcttttcact 120
   ggctgcacct accgct                                                 136